# EUROPEAN PATENT APPLICATION

(11) **EP 4 427 688 A1**
(43) Date of publication of application: **11.09.2024**
(21) Application number: 24162259.6
(22) Date of filing: 08.03.2024
(51) Int. Cl.: A61B 17/68, A61B 17/86

(54) **MULTI-PURPOSE LOCKING AND COMPRESSION SCREW SYSTEM**

(30) Priority: 08.03.2023 US 202363450748 P
(71) Applicant: Stryker European Operations Limited, Carrigtwohill, Co. Cork T45 HX08 (IE)
(72) Inventor: ZANDER, Nils, 24340 Eckernförde (DE); WIELAND, Manfred, 24146 Kiel (DE); KLUEVER, Hendrik, 24232 Schoenkirchen (DE)
(74) Representative: Röthinger, Rainer

(57) **Abstract**

A bone fracture fixation system includes a screw including a head defining an annular groove; and a flexible washer including an outer ring, at least one projection, and a slot at least partially separating the outer ring and the at least one projection, the at least one projection configured to engage the annular groove when the flexible washer is assembled on the screw.

## Description

### CROSS-REFERENCE TO RELATED APPLICATIONS

This application claims the benefit of the filing date of United States Provisional Patent Application No. 63/450,748 filed March 8, 2023, the disclosure of which is hereby incorporated herein by reference.

### BACKGROUND OF THE INVENTION

The present disclosure generally relates to a fracture fixation system, instrumentation, and method of use for fixation of bone fractures of bones such as the femur, tibia, humerus, and radius, including periarticular fractures. More specifically, the present invention encompasses a locking and compression screw system that aids in fixation of screws within fractured bones by applying inter-fragmentary compression while minimizing the possibility of the screws loosening, backing out of the bones, or sinking of the screw head into the cortical bone.

Typical fixation of a fracture of a long bone with a bone plate and/or an intramedullary nail requires making an incision in the tissue, reducing the fracture, inserting an intramedullary nail into the bone , placing a bone plate on the bone, and securing the bone plate to the bone with fixation elements such as screws. The bone plate and the intramedullary nail immobilize the fracture to allow the fracture to heal. Other times, an intramedullary nail may be inserted into the bone and screws may be driven through holes of the intramedullary nail such that no bone plate is used.

Typically, bone plates have a bone-contacting surface and an opposing surface with a plurality of holes or apertures extending between the two surfaces. These holes or apertures may be either threaded (for fixing the plate to the bone with locking screws) or nonthreaded (for fixing the plate to the bone with regular screws) and may be circular or oblong in shape. These holes often correspond to a set of holes formed through intramedullary nail such that screws driven through the bone plate can also be driven through the corresponding holes of the intramedullary nail.

To further secure screws within the fractured bone and to prevent the screws from loosening and/or backing out, or otherwise cutting or diving into the cortex, washers and nuts are often used to secure at least a portion of the screw to an outer surface of cortical bone. Washers may be placed over the shank of the screw such that the screw head contacts the washer as the screw is driven into the bone. Nuts may be placed over the tip of the screw and be driven onto the shank of the screw to contact the opposing cortical region of bone. As such, compressive forces are created between the nut and the washer, and these compressive forces fix the bone fragments.

Current washer and screw devices are not without drawbacks. The rigidity of washers may be difficult to maneuver around nuts and other hardware. Thus, there exists a need for a system that allows for simple insertion of flexible washers and nuts that conform to the shape of the surrounding bone to not interfere with other hardware, such as nuts or bone plates. The present invention addresses this problem and others by providing a fracture fixation system including a screw, a nut, and at least one flexible washer in which there is a secure connection between the screw, nut, and at least one flexible washer.

### BRIEF SUMMARY OF THE INVENTION

In accordance with an aspect of the present disclosure, a method of securing a fractured bone comprises: inserting an intramedullary nail into a medullary canal of the bone; and inserting a bone screw into the bone by driving a tip of the bone screw into the bone and through a first hole of the intramedullary nail, such that a flexible screw washer distinct from the bone screw and surrounding a head of the bone screw conforms to a cortical region of bone and the head of the screw.

In another aspect, the method further comprises drilling a pilot hole through the cortical region of the bone, through the first hole of the intramedullary nail, and through an opposing cortical region of the bone.

In a different aspect, the method further comprises securing a nut to a distal end of the bone screw.

In a further aspect, the securing step includes threading the nut onto a threaded portion of the distal end of the bone screw such that a cannula of the nut extends around the distal end of the bone screw.

In another aspect, the securing step includes securing the nut onto a distal end of the bone screw such that the nut contacts a portion of the opposing cortical region of the bone.

In a different aspect, the securing step includes securing a flexible nut washer distinct from the nut to the nut such that the flexible nut washer conforms to the opposing cortical region of the bone.

In a further aspect, the securing step further includes placing a biasing element of the flexible nut washer into an annular groove of the nut.

In another aspect, the method further comprises driving a tip of a second bone screw through the bone and through a second hole of the intramedullary nail such that a longitudinal axis of a shank of the second bone screw is angularly offset from a longitudinal axis of the shank of the first bone screw.

In a different aspect, the method further comprises attaching the flexible screw washer to an applicator.

In another aspect, the attaching step includes positioning a locator pin of the applicator through a locator hole of the washer such that a central axis of the applicator aligns with a central axis of the flexible washer.

In a different aspect, the method further comprises driving the bone screw through the applicator such that the flexible washer is collected by the head of the screw.

In another aspect, the driving step includes driving the bone screw through the applicator until a biasing element of the flexible washer extends into an annular groove of the head of the screw.

In a further aspect, the biasing element is a radial leaf spring.

According to another aspect of the present disclosure, a method of securing a fractured bone comprises: inserting an intramedullary nail into a medullary canal of the bone; driving a bone screw into the bone such that a tip of the bone screw passes through a first hole of the intramedullary nail; and securing a nut and a flexible nut washer to a distal end of the bone screw and against an opposing cortical region of the bone.

In another aspect, the method further comprises installing a bone plate against the bone such that an inner surface of the bone plate at least partially covers a head of the bone screw that is anchored in a cortical region of the bone within a plane perpendicular to a central axis of the bone screw.

In a different aspect, the method further comprises inserting a linking screw through a plate hole and through a second hole of the intramedullary nail.

In a different aspect, the step of installing the bone plate includes installing the bone plate against the bone such that the inner surface of the bone plate at least partially covers the head of a second bone screw within a plane perpendicular to a central axis of the second bone screw.

In another aspect, a longitudinal axis extending through the first bone screw and a longitudinal axis extending through the second bone screw intersect.

In yet another aspect, the securing step includes threading the nut onto a threaded region of the distal end of the bone screw.

In a further aspect, the securing step includes securing a flexible nut washer distinct from the nut to the nut such that to the flexible nut washer conforms to the opposing cortical region of the bone.

In a different aspect, the securing step further includes placing a spring of the nut washer into a groove in the nut such that spring secures the nut washer to the nut.

In another aspect, the step of installing the bone plate is performed before the step of driving the bone screw; and the step of driving the bone screw includes driving the bone screw through a plate hole of the bone plate and through the first hole of the intramedullary nail.

In another aspect, the bone screw is cannulated.

In a different aspect, the method further comprises inserting a k-wire or guide pin through the cannulated screw.

In a further aspect, the method further comprises guiding the cannulated screw along the k-wire or the guide pin.

According to another aspect of the present disclosure, a bone fracture fixation system comprises: an intramedullary nail; a sleeve; a flexible washer; and a screw including a head defining an annular groove, the annular groove configured to engage the flexible washer when the screw is inserted through the sleeve.

In another aspect, the washer includes an outer ring separated from inner ring portions by at least one channel extending through the washer.

In a different aspect, the channel includes a Y-shaped channel.

In another aspect, the inner ring portions include at least one biasing element configured to engage the annular groove of the head of the screw.

In a different aspect, the biasing element is a radial leaf spring.

In a further aspect, the diameter of the inner ring portions correspond to an outer diameter of a head of the screw.

In another aspect, the washer further comprises an attachment feature for removably attaching to at least one of an end of a sleeve and the head of the screw.

In a different aspect, the attachment feature is a lip.

In yet another aspect, the sleeve includes a flange for removably engaging the lip.

In another aspect, a method of inserting a bone fracture fixation system comprises: inserting an intramedullary nail into a medullary canal of the bone; aligning a targeting guide with the intramedullary nail; attaching an applicator to a distal end of the sleeve and the washer such that the applicator holds the washer; inserting the sleeve through the guide hole of the targeting guide; and inserting the screw through the sleeve such that the head of the screw engages the washer and a shank of the screw passes through a hole of the intramedullary nail.

In a different aspect, the aligning step includes positioning the targeting guide laterally offset from the intramedullary nail such that a longitudinal axis of the intramedullary nail is parallel to a longitudinal axis of the targeting guide.

In another aspect, the inserting the screw step includes inserting the screw through the sleeve to detach the washer from the sleeve and collect the washer against the head of the screw as the screw head advances toward the bone.

In yet another aspect, a bone fracture fixation system comprises: a screw including a head defining an annular groove; and a flexible washer including an outer ring, at least one projection, and a slot at least partially separating the outer ring and the at least one projection, the at least one projection configured to engage the annular groove when the flexible washer is assembled on the screw.

In a different aspect, the at least one projection includes three projections radially spaced around an inner hole of the flexible washer.

In a further aspect, the slot is Y-shaped.

In another aspect, the system further comprises a nut including a head defining an annular groove, the nut configured to extend at least partially over a tip of the screw.

In another aspect, the system further comprises a flexible nut washer distinct from the flexible washer, the flexible nut washer including an outer ring, at least one projection, and a slot at least partially separating the outer ring from the at least one projection, the at least one projection configured to engage the annular groove of the nut.

In a further aspect, at least one projection of the flexible nut washer includes three projections radially spaced around an inner hole of the flexible nut washer.

In a different aspect, the slot includes three Y-shaped slots, each Y-shaped slot corresponding to a projection of the three projections.

### BRIEF DESCRIPTION OF THE DRAWINGS

The features, aspects, and advantages of the present invention will become better understood with regard to the following description, appended claims, and accompanying drawings in which:
FIG. 1 is a perspective view of a fracture fixation system.
FIG. 2 is a side view of a portion of a screw used in the fracture fixation system of FIG. 1.
FIG. 3 is a side view of a portion of a different screw that can be used in the fracture fixation system of FIG. 1.
FIG. 4 is a perspective view of a washer and screw of the fracture fixation system of FIG. 1.
FIG. 5 is a side view of a washer and screw of the fracture fixation system of FIG. 1.
FIG. 6 is a front view of a washer according to one embodiment of the fracture fixation system.
FIG. 7 is a side view of the washer of FIG. 4.
FIG. 8 is a side view the screw of FIG. 2 and the washer of FIG. 8 when the washer is at a distal-most position on the head of the screw.
FIG. 9 is a side view of the screw of FIG. 2 and the washer of FIG. 8 when the washer has traveled to a middle portion of the head of the screw.
FIG. 10 is a side view of the screw of FIG. 2 and the washer of FIG. 8 when the washer is seated within an annular groove of the head of the screw.
FIG. 11 is a perspective view of a tissue protection sleeve and washer according to another embodiment.
FIG. 12 is a perspective view of the tissue protection sleeve and washer of FIG. 11 with a screw inserted within the tissue protection sleeve.
FIG. 13 is a side view of a nut of the fracture fixation system of FIG. 1.
FIG. 14 is a rear perspective view of a nut and nut washer of the fracture fixation system of FIG. 1.
FIG. 15 is a side view of a nut and nut washer of the fracture fixation system of FIG. 1.
FIG. 16 is a side view of the fracture fixation system of FIG. 1 in an implanted configuration.
FIG. 17 is a side view of another embodiment of a fracture fixation system in an implanted configuration.
FIG. 18 is a side view of another embodiment of a fracture fixation system in an implanted configuration.
FIG. 19 is a side view of another embodiment of a fracture fixation system in an implanted configuration.
FIG. 20 is a partially exploded side view of the fracture fixation system of FIG. 1 and its associated insertion instruments.
FIG. 21 is a perspective view of an applicator for attaching a nut of the fracture fixation system of FIG. 1.
FIG. 22 is a perspective view of an applicator attached to a nut of the fracture fixation system of FIG. 1.
FIG. 23 is a perspective view of a tissue protection sleeve of the associated insertion instruments of FIG. 20.
FIG. 24 is a perspective view of a tissue protection sleeve, applicator, and washer of the associated insertion instruments of FIG. 20.
FIG. 25 is a perspective view of a tissue protection sleeve, applicator, washer, and screw of the associated insertion instruments of FIG. 20.

### DETAILED DESCRIPTION

As used herein, when referring to bones or other parts of the body, the term "proximal" means closer to an operator and the term "distal" means further away from the operator. The term "anterior" means toward the front of the patient's body and the term "posterior" means toward the back of the patient's body. The term "inferior" means toward the patient's feet and the term "superior" means towards the patient's head. The term "medial" means toward the midline of the body and the term "lateral" means away from the midline of the body.

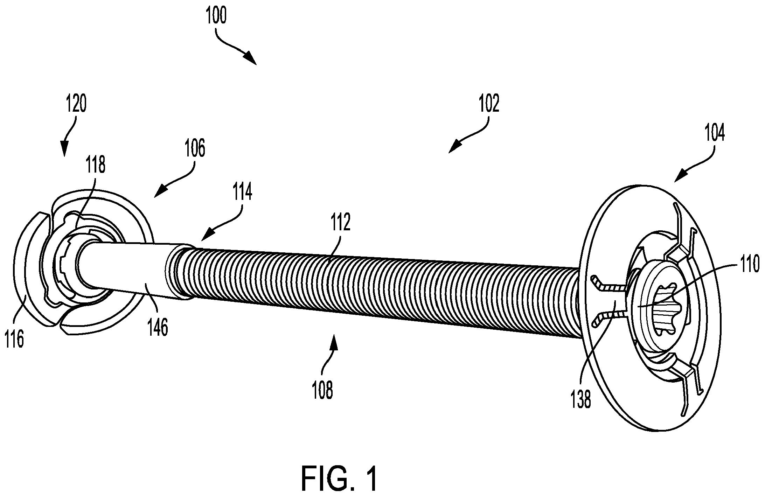

FIG. 1 illustrates a perspective view of a fracture fixation system 100 for securing a fractured femur. Although the embodiments described and depicted herein illustrate a femur, it is envisioned that the fracture fixation system 100 may be compatible with other bones, such as the tibia, humerus, and other long bones. Fracture fixation system 100 includes a screw 102 extending along a longitudinal axis. A separate and distinct washer 104 is configured to engage with and surrounds at least a portion of the head 110 of screw 102, and likewise a separate and distinct nut 106 is configured to engage with and surround at least a portion of a tip 114 of screw 102. Various thread patterns 108 may be formed along a shank 112 of screw 102 to allow screw 102 to purchase within a bone and to allow nut 106 to engage screw 102. A nut washer 120, which is separate and distinct from washer 104, surrounds and engages at least a portion of nut 106.

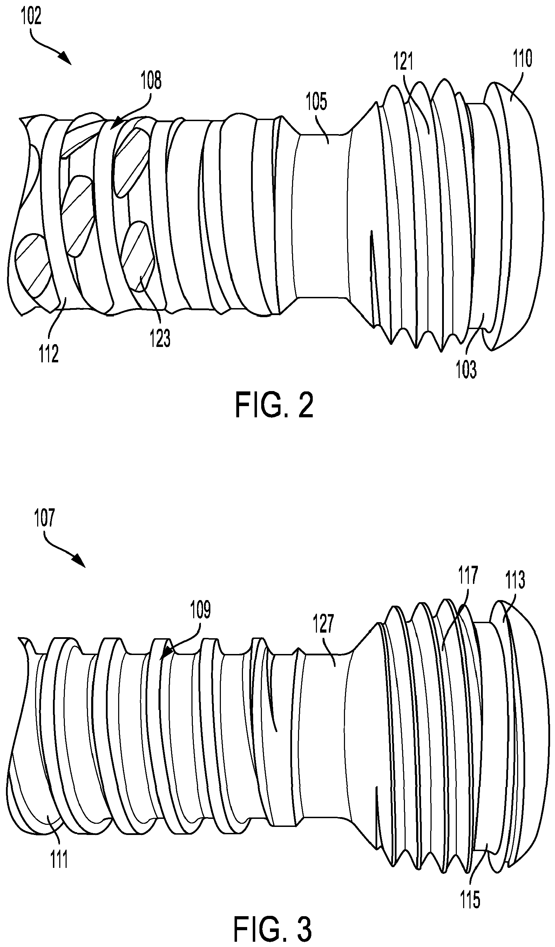

FIGS. 2-3 illustrate portions of different screw embodiments that can be implemented with fracture fixation system 100. Screw 102, depicted in FIG. 2, extends along a longitudinal axis and includes a head 110 at a proximal end, a neck 105 adjacent to head 110, and a shank 112 adjacent neck 105. An annular groove 103 extends around an outer circumference of head 110 such that a washer may sit within annular groove 113 and not interfere with threads 121 disposed along screw head 110. Annular groove 113 is preferably positioned toward a proximate end of head 110 such that threads 121 positioned at a more distal portion of head 110 may have sufficient purchase within a bone. Various thread patterns 108 may be formed into shank 112. As depicted, thread pattern 108 includes a plurality of notches 123 spaced circumferentially through the threads of thread pattern 108. Notches 123 are configured to provide additional purchase within bone and may be advantageous for certain fractures. Such a design is disclosed in U.S. Patent No. 11,116,557, the entire disclosure of which is hereby incorporated herein by reference

FIG. 3 illustrates another embodiment of a screw 107. Screw 107 includes a screw head 113 including an annular groove 115 positioned proximally and threads 117 positioned distal of annular groove 115. A neck 127 extends from head 113 and tapers toward shank 111. Neck 127 is preferably unthreaded but may be threaded in alternative embodiments. A thread pattern 109 is formed into shank 111 to allow shank 111 to purchase within a bone. As depicted, thread pattern 109 has a uniform thread pitch that is void of any notches or other fixation features. Threads 117 of head 113 are configured to engage cortical bone to secure screw 107 within the bone. A washer is configured to sit within annular groove 115 such that the washer is secured between a proximal portion of head 113 and threads 117 positioned at a distal portion of head 113. This position secures a washer to head 113 without interfering with threads 117. Annular groove 115 is similar to annular groove 103 such that the same washer 104 may be implemented with both screws.

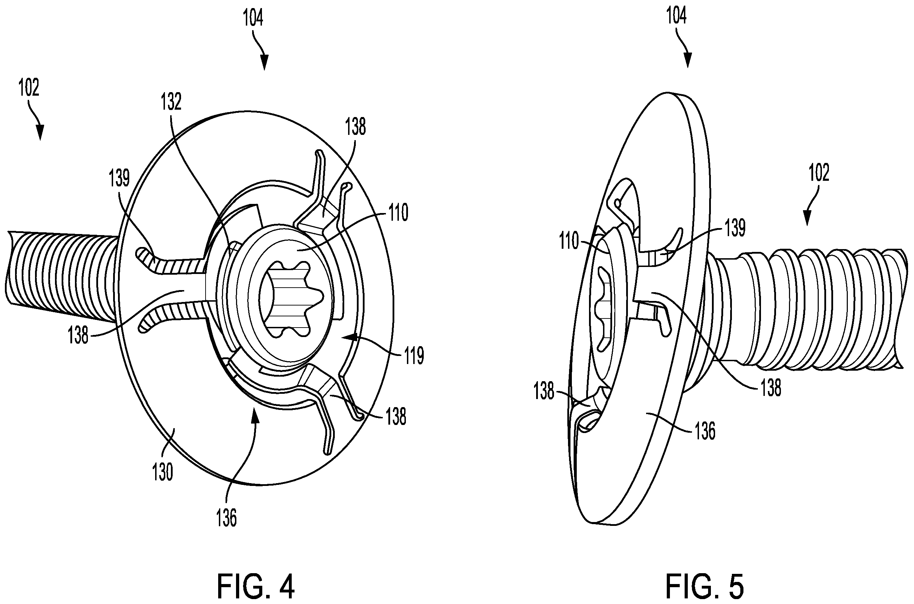

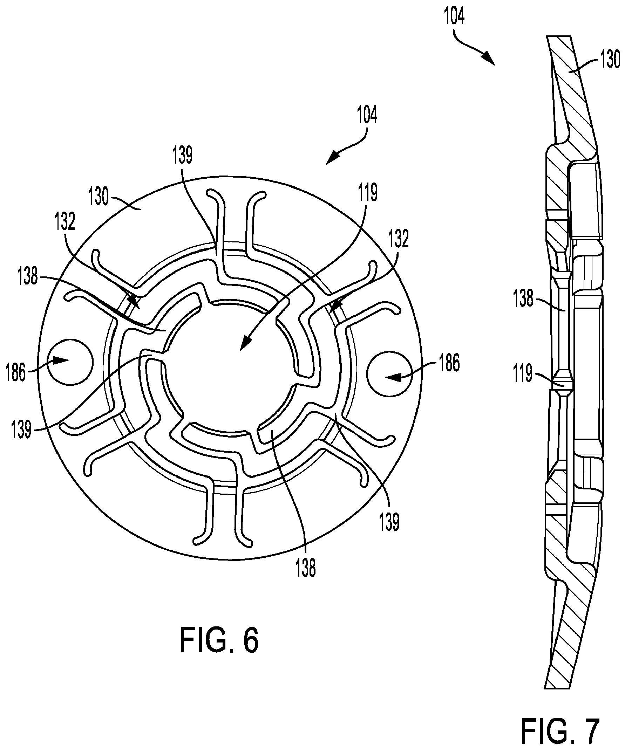

FIGS. 4-7 illustrate a washer 104. Washer 104 is flexible and defines a generally circular or annular shape when it is in a relaxed configuration. As such, washer 104 defines an outer ring 130 having an outer diameter and inner ring portions 132 that define a central hole 119 and comprise various attachment features. Further, washer 104 may have various attachment holes 186, such as those illustrated in FIG. 6, for attaching to an applicator or other insertion tool. The diameter of hole 119 is slightly larger than the diameter of shank 112 of screw 102 to allow screw 102 to pass through hole 119. Washer 104 has a substantially uniform thickness across its surface to allow for a uniform distribution of forces in the implanted configuration. Washers 104 may be made of any implant material having elastic properties. Such materials include stainless steel, cobalt-chrome, titanium, titanium alloys, high strength polymers such as polyetheretherketone (PEEK), and fiber-reinforced polymers.

Hole 119 of washer 104 defines an attachment hub 136 for attaching washer 104 to a screw head 110. As such, at least one attachment feature, such as arm 138, may extend radially inward from the outer ring 130 toward the center of hole 119. Arm 138 may be a radial leaf spring or other attachment feature configured to flex and bias inwards toward the center of hole 119 such that the biasing force holds washer 104 to screw head 110. As depicted in FIG. 4, arm 138 includes three arms positioned approximately 120° away from each other around the circumference of head 110. In the embodiment of FIG. 6, six arms 138 are included. Thus, any number of arms may be implemented to secure washer 104 to screw head 110. Arms 138 may be substantially L-shaped as depicted, or may be another shape configured to engage annular groove 103 of screw head 110. The thickness of arms 138 corresponds to the thickness of annular groove 103 such that arms 138 may sit within annular groove 103 to secure washer 104 to screw 102. Arms 138 may be defined between various grooves or cutouts 139 in outer ring 130. As depicted, each arm 138 includes Y-shaped cutouts 139 extending radially outward from each arm 138 into the outer ring 130. Cutouts 139 form gaps around the L-shaped body of arm 138. Such gaps may be arced or contoured to extend radially around the perimeter of hole 119. Further, arms 138 may be angled as shown in FIG. 4 such that head 110 of screw 102 sits flush or substantially flush with outer ring 130 of washer 104. With this configuration, a screw engaged with a washer can be implanted into bone such that the screw head is nearly flush with the outer surface of cortical bone and the washer sits slightly proud of the screw head. Thus, the screw head can fully engage the underlying cortical bone and experience the benefits of having a washer. In other embodiments of washer 138, arm 138 may further include a lip, raised edge portion, or other securing feature for facilitating attachment with a sleeve 140.

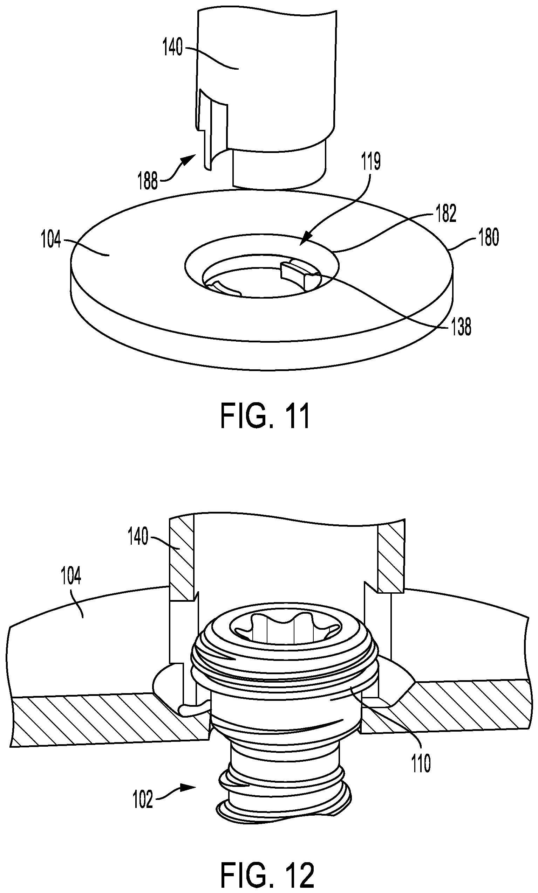

In another embodiment of washer 104b depicted in FIGS. 11-12, washer 104b may define a body having an outer diameter 180 and an inner diameter 182, inner diameter 182 forming hole 119. The inner diameter is larger than the diameter of shank 112 of screw 102 to allow screw 102 to pass through hole 119. Such a washer 104b may have a substantially uniform thickness across its surface to allow for uniform distribution of forces in the implanted configuration. Arms 138 may extend inward from the walls defining hole 119 such that arms 138 engage with a screw head 110. Such arms 138 may be radial leaf springs, helical coil springs, or the like. In other embodiments, arms 138 may include other attachment features, such as lips or grooves. Each of these attachment features may facilitate attachment between a washer 104 and screw head 110.

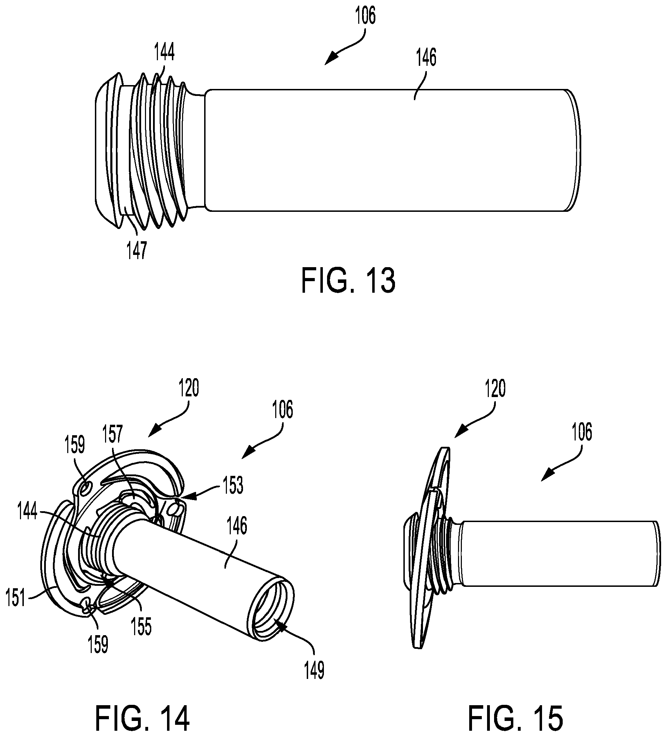

FIGS. 13-15 illustrate a nut 106 according to one embodiment of the present invention. Nut 108 includes an elongate cylindrical portion 146 and a head portion 144 adjacent to the cylindrical portion 146. Head 146 is threaded such that the threads can engage cortical bone. An annular groove 147 extends around the perimeter of head 146 such that a nut washer 120, which is separate and distinct from washer 104, can be secured to nut 106. Such an annular groove 147 is similar to annular grooves 103 and 115, and thus will not be described fully again for sake of brevity. Nut 106 defines a cannula 149 that may be at least partially internally threaded to engage threads of screw 102. Cannula 149 may extend throughout the entire length or partially through the length of cylindrical portion 146. In alternative embodiments, nut 106 may include a cylindrical portion 146 and a conical portion between the cylindrical portion and a head portion. Such a conical portion may be frustoconical to provide a surface for nut washer engagement.

FIG. 14-15 illustrate a nut washer 120 that is separate and distinct from washer 104. Nut washer 120 differs slightly from washer 104, although both washer 104 and nut washer 120 may be interchangeable. Nut washer 120 is generally circular in a relaxed configuration and may flex and change shape under stress such that it matches the shape of the underlying bone. Nut washer 120 includes outer ring portions 151 extending around an outer perimeter of nut washer 120. Outer ring portions 151 are flexible and are separated by several channels 153. Channels 153 extend inward from a point on the outer perimeter of nut washer 120 to a central hole 155. Central hole 155 is configured to receive the head 144 of nut 106. Channels 153 further define at least one attachment member, such as a spring 157. As depicted, nut washer 120 includes three outer ring portions 151, three channels 153, and three springs 157. In other embodiments, a different number of nut washer components may be implemented.

Springs 157 are configured to bias inward toward the center of central hole 155. In use, springs 157 at least partially wrap around and extend into annular groove 147 of nut 106 to prevent nut washer 120 from detaching from nut 106. Thus, the thickness of springs 157 may correspond to the thickness of annular groove 147 in order to stay seated without slipping out of annular groove 147. Further, springs 157 may be angled downward to prevent the screw head 144 of nut 106 from extending proud to an outer proximal surface of nut washer 120. Nut washer 120 may further include other features such as an outer lip or various other cutouts/configurations that allow outer portions 151 to bend and flex relative to each other while seated on nut 106 in the implanted configuration.

Nut washer 120 includes at least one locator hole 159 configured to mate with a locator pin of an applicator (described herein below). Each locator hole 159 may be positioned within each outer portion 151. Alternatively, not all outer portions 151 may have a locator hole 159, or one outer portion 151 may have multiple locator holes 159.

In alternative embodiments of nut washer 120, nut washer 120 may look and operate substantially similar to the alternative embodiment of washer 104 described above and illustrated in FIGS. 11-12, although instead of engaging an annular groove within screw head 110, nut washer 120 may engage an annular groove within nut 106.

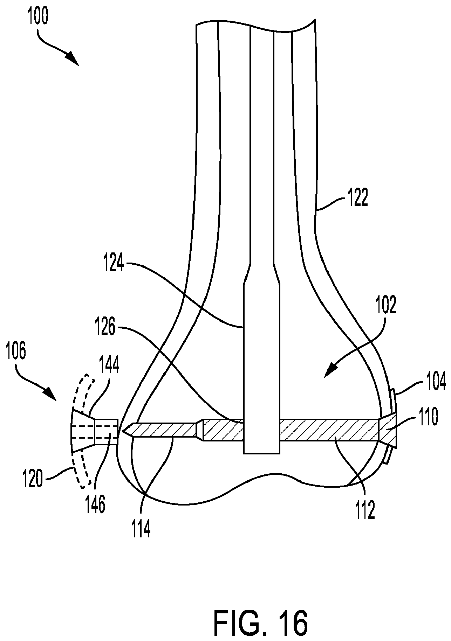

FIG. 16 depicts an embodiment of fracture fixation system 100 implanted into a femur 122. In such an embodiment, fracture fixation system 100 additionally includes an elongate intramedullary nail 124 with at least one hole 126. Shank 112 of screw 102 extends through hole 126 such that head 110 is anchored into a cortical region of bone and tip 114 is initially anchored into an opposing cortical region of bone. In the implanted configuration, washer 104 surrounds at least a portion of head 110 and contacts the cortical region of bone. A nut washer 120 surrounds at least a portion of nut 106 and nut 106 surrounds at least a portion of tip 114 and anchors into the opposing cortical region of bone.

Continuing with this embodiment, intramedullary nail 124 defines and extends along a longitudinal axis that is substantially parallel to a longitudinal axis of the long bone intramedullary nail 124 is implanted within. Intramedullary nail 124 may have a circular cross-section along its axis and be inserted into a medullary canal of a patient via common insertion methods. The medullary canal contains marrow and is circumferentially encased by cortex which provides a dense outer surface that fasteners, washers, and nuts can engage with.

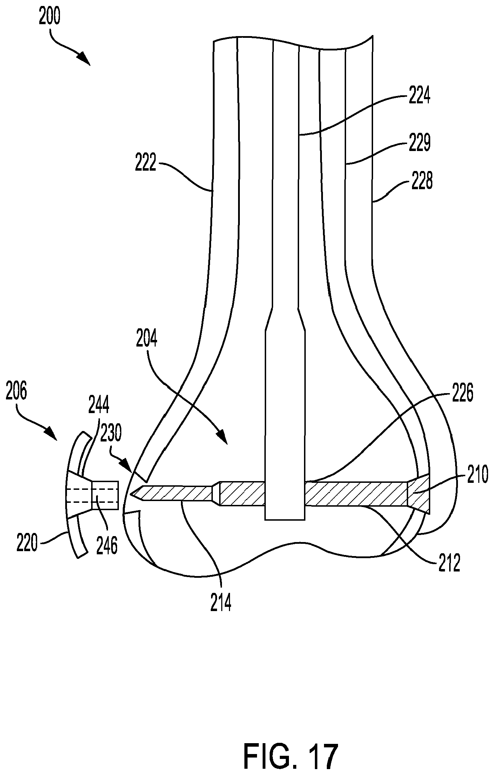

One or more holes 126 are defined by and extend transversely through intramedullary nail 124. The diameters of holes 126 may correspond to the diameter of various fasteners that will be driven therethrough. Accordingly, the diameters of holes 126 may vary from one hole to the next depending on factors such as the intended fastener size, the patient's bone structure, and/or the location of the fracture. Each hole 126 defines an axis extending therethrough. Each hole axis may be parallel to the other hole axes or may be angularly offset therefrom. As such, different fasteners may extend through holes 126 at different angles relative to each other. In some circumstances, holes 126 are angled such that fasteners extending therethrough have a trajectory that passes next to a bone plate (in embodiments where a bone plate is implemented and a screw is inserted below the bone plate, such as the embodiment of FIG. 17) on either the anterior or posterior side. Holes 126 may have internal threading or other retainment features to secure fasteners extending therein.

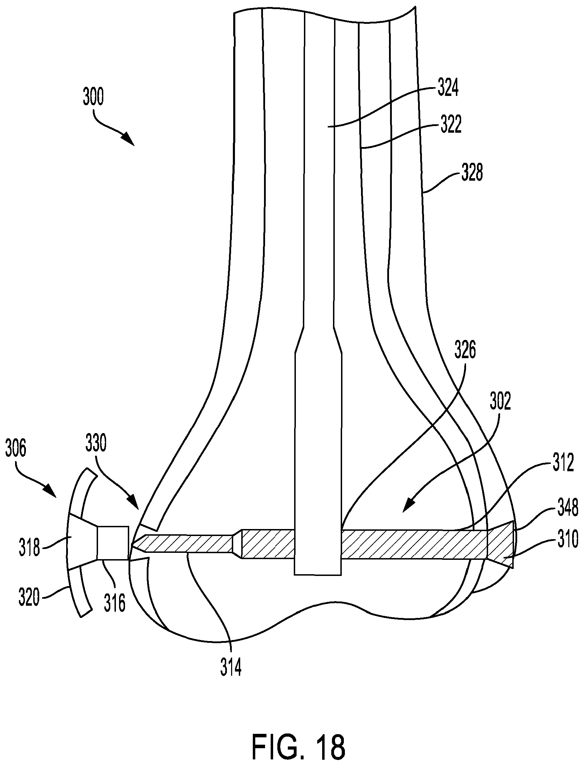

Screws 102 extending through holes 126 of intramedullary nail 124 may be any screws known in the art, such as plunger screws, linking screws, or unlinked screws. Screws 102 may have various thread patterns and head, shank, and tip designs depending on particular applications. For example, FIG. 15 depicts a plunger screw 202 in which head 210 is sunk to a location at or beneath an inner surface of bone plate 228 that sits flush against the bone surface. Further disclosure related to this configuration can be found in U.S. Patent App. No. 63/334,883, the disclosure of which is incorporated by reference herein. FIG. 18 depicts a linked screw 302 in which head 310 is anchored into bone plate 328. Further, screws 102 may have various thread patterns such as right-hand threads, left-hand threads, or a mixture of both. Thus, an operator may select various screws based on unique characteristics of a patient's fracture. Screw 102 may have a continuous diameter across its shank 112 or it may have a diameter that varies across the shank 112 such as the distally tapering diameter shown in FIG. 16.

FIG. 17 illustrates another embodiment of a fracture fixation system 200. In this embodiment, fracture fixation system 200 is similar to fracture fixation system 100, and therefore like elements are referred to with similar numerals within the 200-series of numbers. In addition to the components of fracture fixation system 100, fracture fixation system 200 includes a bone plate 228. Bone plate 228 is elongated and extends from a superior end to an inferior end. An inner surface 229 of bone plate 228 is configured to face and contact the bone and an opposing outer surface of bone plate 228 is configured to face away from the bone, e.g., in a lateral direction. Inner surface 229 may be substantially concave or otherwise shaped to contour to the underlying bone surface. The curvature of the inner surface 229 may also be configured to initially define a gap between the inner surface and the underlying femur 222. The inner surface 229 may then flex such that the gap is closed when fasteners are driven though bone plate 228.

Continuing with the embodiment of FIG. 17, inner surface 229 of bone plate 228 may be substantially smooth and lack slots, cutouts, or other regions (aside from any separate screw holes therethrough) that could accept a head of an underlying fastener. Such a smooth inner surface is configured to sit flush or substantially flush with the underlying bone. Screws 202, such as plunger screws, are countersunk and anchored into the underlying cortical bone and may sit flush with the outer cortical bone surface or be sunk beneath the cortical surface to avoid interfering with the seating of the bone plate 228 on the bone. In this implanted configuration, inner surface 229 of bone plate 228 may cover an aperture or a portion of an aperture in bone defined by the screw heads 210 driven therein.

Bone plate 228 preferably defines at least one aperture (as shown in FIGS. 18-19) extending transverse to the longitudinal axis of the plate 227. This aperture includes a pattern of apertures across bone plate 228. Different hole patterns may be implemented for different bone plates, fracture types, and applications through a patient's body. The aperture is configured to receive a fastener, such as bone screw 202, therethrough and may also be configured to receive other attachment features, such as a sleeve 240, insertion tool, or a pin from an aiming block (not shown) therethrough. The aperture may further have contouring along its internal surface to engage with at least one of the head of a fastener driven therethrough or an attachment feature of an insertion instrument or sleeve 240. Such contouring may allow for fastener insertion at variable angles while maintaining a strong connection with the head of the fastener. This variable angle locking concept relies on a multi-point form-/friction-fit fixation between plate and screw. This allows for a sufficient interference fit between plate and screw, thus preventing an unintended screw back-out in-vivo. Further disclosure related to the configuration is found in U.S. Patent No. 11,039,865, the disclosure of which is incorporated by reference herein.

The aperture may be threaded or unthreaded. If it is threaded, then a fastener driven therein may only be inserted along a fixed axis, i.e., the central longitudinal axis of the aperture. Alternatively, if the aperture is unthreaded, then a fastener driven therein may be driven along multiple axes at variable angles. The aperture may be circular or elliptic. In some embodiments, an elliptic aperture (not shown) may accept multiple fasteners, each configured for a different purpose. For example, one fastener may lock the bone plate to a first bone fragment and another fastener may compress a second bone fragment toward the first bone fragment. In other embodiments, a single fastener may be inserted at any one of multiple locations along the elliptic aperture or slot.

Continuing with the embodiment of FIG. 17, fracture fixation system 200 further includes a nut 206 and a nut washer 220 configured to secure a tip 214 of screw 204 to bone and spread the forces around the bone such that nut 206 does not sink or move in the implanted configuration. Nut 206 and nut washer 220 are similar to nut 106 and nut washer 120, and thus will not be described fully again for sake of brevity.

FIG. 18 illustrates another embodiment of a fracture fixation system 300. In this embodiment, fracture fixation system 300 is similar to fracture fixation system 100 and 200, and therefore like elements are referred to with similar numerals within the 300-series of numbers. Fracture fixation system 300 includes an intramedullary nail 324 with at least one hole 326 extending therethrough. A screw 302 extends into hole 326 such that a screw head 310 is anchored in an aperture 348 of bone plate 328 and a tip 314 of screw 302 is at least initially anchored in a cortical region of bone. A nut 306 secures distal tip 314 to the cortical region of bone, and a flexible nut washer 320 provides compressive force from the nut 306 against the cortical bone. Because screw head 310 is anchored in an aperture 348 of bone plate 328, it may not require a washer as the compressive forces are spread from the screw head 310 throughout bone plate 328 without a washer.

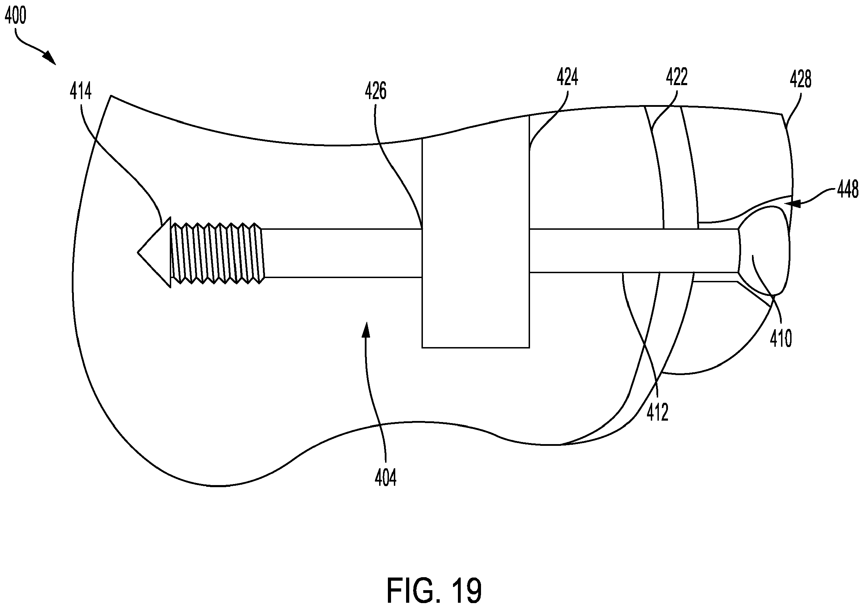

FIG. 19 depicts another embodiment of fracture fixation system 400. Fracture fixation system 400 includes an intramedullary nail 424, a cannulated screw 402 having a head 410 secured in an aperture (not shown) of a bone plate 428, and a cutting portion 414 at the distal tip of screw 402. Cutting portion 428 extends through a hole 426 of intramedullary nail 424 but does not extend into an opposing cortical region of bone. Cutting tip 414 includes cutting flutes that facilitate both implantation and removal of screw 402 without the drilling of pilot holes. Such cutting flutes may have any flute design known in the art and may work in conjunction with a cutting tip configured to puncture cortical bone. As the cutting flutes of cutting tip 414 engage with the surrounding bone while screw 402 is rotated, a compressive force is created along the length of screw 402 to secure it in place.

The cannulation of screw 402 (not shown) allows a k-wire or other guidewire (not shown) to be passed through screw 402 to guide the insertion of screw 402 without predrilling pilot holes. As such, the k-wire may be passed through an aperture in bone plate 428 and through a hole 426 in intramedullary nail 424 before screw 402 is translated along the k-wire while simultaneously cutting the bone 422 to secure screw 402 therein.

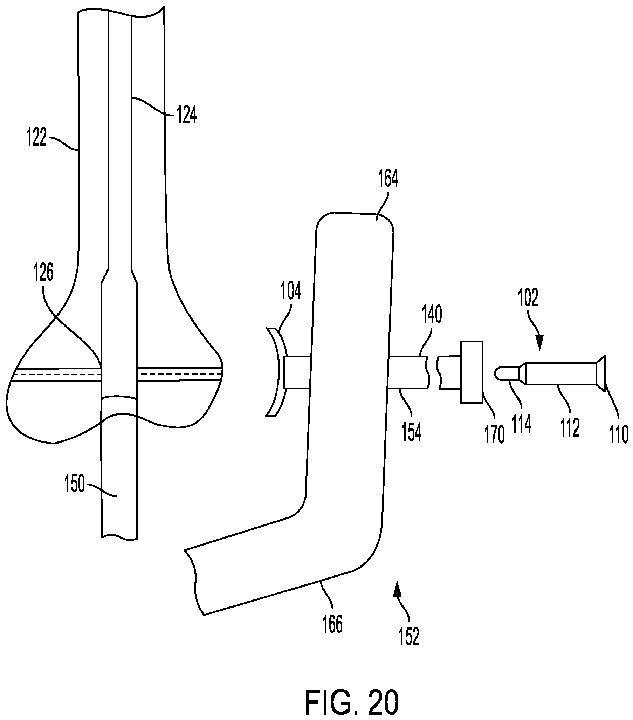

FIG. 20 illustrates a partially exploded view of the insertion instruments for inserting fracture fixation system 100. In the embodiment depicted, fracture fixation system 100 includes an intramedullary nail 124, screw 102 configured to extend through a hole 126 of intramedullary nail 124, and a washer 104. The insertion instruments include a sleeve 140, a targeting arm 152, and an applicator 170. Each of these components will be described in detail below.

FIG. 20 depicts a targeting arm 152 used to aid in insertion of fracture fixation system 100. Targeting arm 152 includes an elongate targeting portion 164 and an elongate connection portion 166 attached to targeting portion 166. Targeting portion 166 defines and extends along a longitudinal axis that is parallel or substantially parallel to the longitudinal axis of the underlying bone 122 and intramedullary nail 124. Targeting portion 164 includes a hole pattern that corresponds with a hole pattern of the intramedullary nail 126. As such, an operator may pre-select certain targeting arms 152 that corresponds to a selected intramedullary nail 124 to ensure proper alignment of all hole axes of fracture fixation system 100.

Targeting portion 164 may have a substantially rectangular cross-section or may have another cross-sectional shape known in the art. Targeting portion 164 may be integrally formed with connection portion 166 or each component may be distinct and assembled during use. Connection portion 166 may have the same cross-sectional shape as targeting portion 164 or a different shape depending on the particular application. Connection portion 164 connects to an intramedullary nail adaptor 150 that extends from intramedullary nail 124 along the same longitudinal axis and out of the bone 122. Connection portion 164 may attach to adapter 150 via fasteners, integral connections, jaws, or the like to orient targeting portion 164 such that it extends substantially parallel to intramedullary nail 124 to allow fasteners to be driven towards holes 126 of intramedullary nail 124.

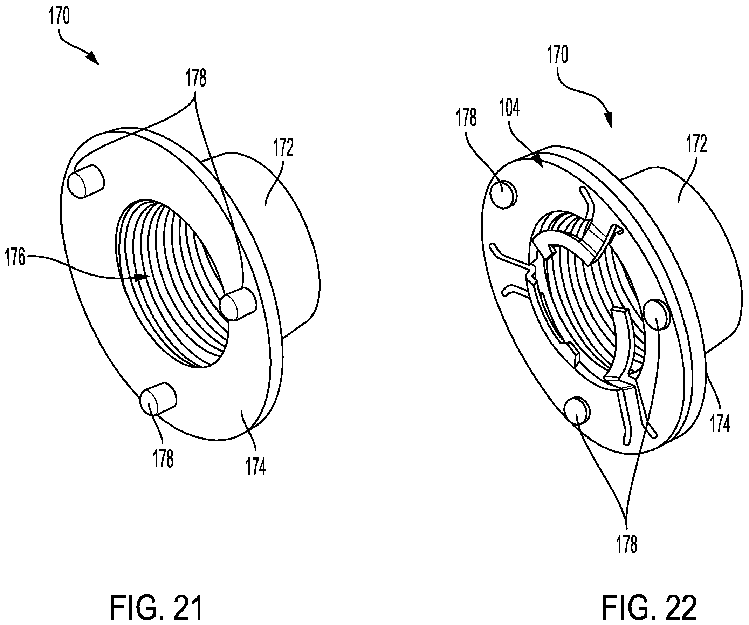

FIGS. 21-22 depict an applicator 170 according to one embodiment. Applicator 170 includes a proximal cylindrical portion 172, an engagement portion 174 connected at the distal end of cylindrical portion 172, and a threaded cannula 176 extending longitudinally within the cylindrical portion 172 and engagement portion 174. Engagement portion 174 is ring shaped and has a diameter that corresponds to a diameter of a washer. Engagement portion 174 includes at least one locator pin 178 extending distally from the surface of engagement portion 174. As depicted, locator pins 178 are cylindrical and rounded at the distal end. In alternative embodiments, locator pins 178 may be square or other shapes that correspond to an attachment hole or feature of a washer.

Cylindrical portion 172 has a smaller diameter than engagement portion 174 but a larger diameter than sleeve 140 such that sleeve 140 extends within cylindrical portion 172 and threads 156 of sleeve 140 engage threaded cannula 176 of applicator 170. The diameter of threaded cannula 176 is slightly larger than the diameter of the threads of the screw heads driven therethrough such that a washer doesn't collect onto the threads, but rather into the annular groove within the screw head.

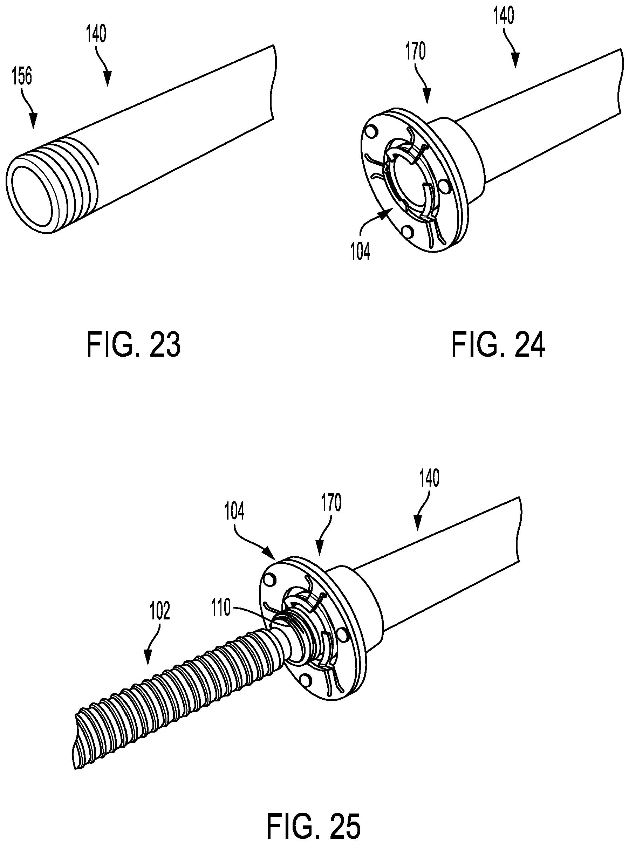

FIGS. 23-25 illustrate a sleeve 140. Sleeve 140 defines and extends along a longitudinal axis from a proximal end to a distal end. Sleeve 140 is cylindrical and has a continuous diameter across its length. This diameter corresponds to a diameter of a screw 102 that is passed through sleeve 140 without being substantially larger than the diameter of screw 102 so that damage to the surrounding tissue is minimized. A proximal end of sleeve 140 may have a greater diameter than the body of sleeve 140 to allow sleeve 140 to engage a targeting arm 152 without passing through a hole 154 of targeting arm 152. In other embodiments, the diameter of sleeve 140 may vary according to the diameter of the screw that is driven therethrough or based on various factors. Sleeve 140 preferably has a length that is longer than a screw that will be driven through sleeve 140 so that the surrounding tissue is protected during insertion of fracture fixation system 100.

Distal end of sleeve 140 includes threads 156 for attaching to the applicator 170, as depicted in FIGS. 24-25. Threads 156 correspond to inner threads 176 of applicator 170, such that washer 102 can be secured to applicator 170 and then screw 102 can be driven through sleeve 140 to collect washer 102 within an annular groove 103, 115 of head 110, 113. The diameter of threads 156 is slightly larger than the diameter of the threads disposed within the screw heads driven therethrough so the washer 102 does not catch on the threads before seating into annular groove 103, 115.

In alternative embodiments, such as those depicted in FIGS. 11-12, sleeve 140 may include a cutout 188 at its distal end rather than threads. Such a cutout 188 is designed to bisect distal end of sleeve 140 in order for the distal end to flex and extend around a screw head 110. The distal end of sleeve 140 may then contact arms 138 or hole 119 to align a central axis of sleeve 140 with a central axis of washer 104 such that screws 102 can be readily driven through sleeve 140 and collect washer 104.

A method of inserting fracture fixation system 100 is described herein. Although the methods described herein are implemented with a femur, it is envisioned that these methods may be applied to other bones throughout a patient's body. An intramedullary nail 124 is first inserted into the patient's femur using standard insertion techniques. An operator may then select an appropriate screw 102, washer 104, nut 106, nut washer 120, and various insertion instruments based on the selected intramedullary nail and the patient's unique fracture. An intramedullary nail adapter 150 may be attached to intramedullary nail 124 such that adaptor 150 extends longitudinally from the patient's body on the same axis as intramedullary nail 124. Pilot holes may be drilled through hole patterns of targeting arm 152, the underlying femur 122, and through hole 126 of intramedullary nail 124 to provide guidance for screw 102 when it is driven into the bone. In embodiments where plunger screws are implemented, an operator may also drill a countersunk recess at the point where the pilot hole punctures the cortical bone.

Sleeve 140 may then be inserted into targeting portion 164. Once the distal tip of sleeve 140 is inserted through targeting portion 164, an applicator 170 is threaded onto the distal end threads 156 of sleeve 140. A washer 104 is then secured to the applicator 170 by pushing locator pins 178 of applicator 170 through corresponding locator holes of washer 104. This connection between applicator 170 and washer 104 may result in a tactile response, such as a "click" or "snap," which indicates to an operator that washer 104 is secured to applicator 170. Alternatively, washer 104 may be pre-loaded onto applicator 170.

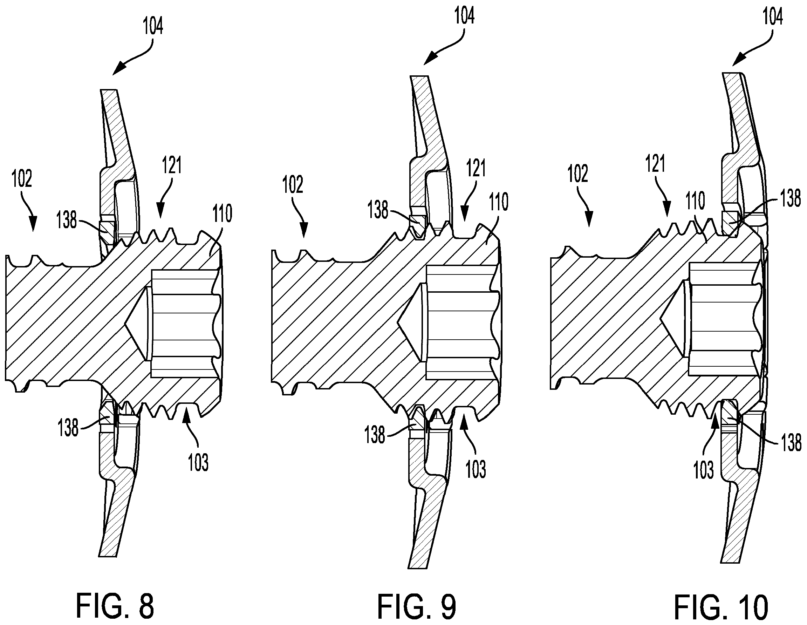

With targeting arm 152 in place, screw 102 is longitudinally inserted into sleeve 140 such that the tip 114 of screw 102 travels distally through sleeve 140 and through hole 119 in washer 104. An operator may rotate screw 102 using a manual driver, drill, or other insertion tool known in the art such that the tip 114 of screw punctures the cortical bone and the threads purchase within the underlying bone. Sleeve 140 protects the surrounding tissue from the threads of screw 102 and from shavings or other debris created as screw 102 is drilled into bone, and also acts as a drill guide. As screw head 110 passes through sleeve 140 to a point distal of targeting portion 164, it begins to collect washer 104 on screw head 110. Washer 104 travels proximally over the threads 121 of screw head 110 until arms 138 of washer 104 deform and bias inward into annular groove 103. Due to the flexibility of washer 104 and arms 138, the washer 104 may elicit a tactile response, such as a "click," as it passes over each thread 121 and once it is seated in annular groove 103. In this way, washer 104 distributes a compressive force on the portion of bone surrounding the screw hole to firmly secure head 110 of screw 102 in place and to enhance the compressive design of fracture fixation system 100. The flexibility of washer 104 permits this even distribution of forces and also allows washer 104 to confirm closely with the surrounding bone surface to minimize its height outside of the bone. Such a seating of washer 104 onto screw head 110 is illustrated in FIGS. 8-10.

In an embodiment that implements a nut washer 120 and a nut 106, the same method steps described above may be taken. Once tip 114 of screw 102 is accessible at the opposing cortical region of bone through the pre-drilled hole, an operator may first insert a nut washer 120 over the head 144 of nut 106 until a spring 157 seats within annular groove 147 of nut 106. This seating may result in a tactile response similar to the responses described above for washer 104 and annular groove 103. Once nut washer 120 is secured in place over nut head 144, cannula 149 of cylindrical portion 146 of nut 106 is placed over screw tip 114 such that screw tip 114 extends into cannula 149. Nut 106 may then be threaded onto tip 114 such that the threads of screw 102 engage the threads of cannula 149. Nut 106 may then be securely tightened either by hand, by a manual tool, or by an automatic tool such that it engages cortical bone and flexible nut washer 120 conforms to both bone and nut 106.

Another method of inserting a fracture fixation system 200 is similar to the method of inserting fracture fixation system 100, and thus like numerals will be referred to in the 200-series of numbers. Fracture fixation system 200 includes a bone plate 228 placed over the top of plunger screw head 210. Similar insertion methods are used to the method of inserting fracture fixation system 100, albeit no washer is placed around the head of screw 202. Before screw 202 is driven into bone 222, a countersunk recess is formed in the bone. Such a countersunk recess is configured to receive screw head 210 when it is driven into bone such that screw head 210 sits flush or substantially flush with the surface of bone 222. Once screw 202 is securely in place, an inner surface of bone plate 229 may be placed against the bone and over the top of the countersunk screw head 210. Additional screws may then be passed through holes of bone plate 228 into the underlying bone and into the holes of intramedullary nail to secure bone plate 228 to bone. A nut 206 and nut washer 220 may be placed on the distal tip 214 of screw 202 using methods similar to those described herein.

Another method of inserting a fracture fixation system 300 is similar to the method of inserting fracture fixation system 100 and 200, and thus like numerals will be referred to in the 300-series of numbers. Fracture fixation system 300 utilizes linking screws 302 that link bone plate 328 to intramedullary nail 324. This method begins with the insertion of an intramedullary nail 324, an intramedullary nail adapter 150, and a targeting arm 152. Once targeting arm 152 is in place, a bone plate 328 is selected based on factors such as location and type of bone fracture. Once selected, bone plate 328 is placed against femur322 such that apertures 348 of bone plate 328 align with holes 154 of targeting arm 152 and holes 326 of intramedullary nail 324. Of course, some of apertures 348 may not align with nail 324 to permit additional bone fixation. Depending on whether cannulated screws or noncanulated screws are used, pilot holes may be drilled through apertures 348 of bone plate 328 and through holes 326 of intramedullary nail 324.

Continuing with this method, sleeve 140 is placed through a hole 154 of targeting portion 164 and into a hole of bone plate 348 that aligns with a hole 326 of intramedullary nail 324. This method does not necessarily implement washers for the head of the screw. As such, a distal tip of sleeve 140 may be placed directly into hole 348 of bone plate 328 to guide screw head 310 directly into that desired hole 348. Once in place, at least one nut washer 320 and at least one nut 306 may be secured to the tip 314 of screw 302, if desired. Sleeve 140 may still be used as a drill guide in this or any other method whether or not washer 104 is employed.

Another method of inserting a fracture fixation system 400 is similar to the method of inserting fracture fixation system 300, and thus like numerals will be referred to in the 400-series of numbers. Fracture fixation system 400 utilizes linking screws 402 that link bone plate 428 to intramedullary nail 424, albeit screws 402 are cannulated. The same insertion steps as the method of inserting fracture fixation system 300 apply to fracture system 400. In addition to the method steps of inserting fracture fixation system 300, an operator may pass a k-wire (not shown) through cannulation of screw 402, an aperture 448 of bone plate 428, femur 422, and hole 426 of intramedullary nail 424. Screw 402 is then inserted using the insertion methods described above while being passed along a k-wire. As screw 402 is rotated into position, cutting flutes of distal tip 414 cut into and purchase with bone 422. Once screw 402 is in place, the k-wire may be retracted proximally from screw 102.

Each component of the fracture fixation systems described herein may be provided in a kit. The kit may have different bone plates for different fracture types, different sizes and types of screws (e.g., cannulated v. non-cannulated), different size sleeves, and various numbers of washers and nuts. This kit may allow an operator to select from a range of component sizes to best suit each individual patient.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to a further advantage. Any reference signs in the claims should not be construed as limiting the scope.

## Claims

1. A bone fracture fixation system comprising:
a screw including a head defining an annular groove; and
a flexible washer including an outer ring, at least one projection, and a slot at least partially separating the outer ring and the at least one projection, the at least one projection configured to engage the annular groove when the flexible washer is assembled on the screw.

2. The system of claim 1, wherein the slot is Y-shaped.

3. The system of claim 1 or 2, wherein the at least one projection includes three projections radially spaced around an inner hole of the flexible washer.

4. The system of claim 3, wherein the slot includes three Y-shaped slots, each of the slots extending into the outer ring and corresponding to the three projections.

5. The system of claim 4, further comprising three arcuate grooves defined between the three projections.

6. The system of any of the preceding claims, wherein each of the at least one projection is configured to be biased inwards against the annular groove to retain the flexible washer to the head of the screw when the flexible washer is assembled on the screw.

7. The system of any of the preceding claims, further comprising a nut including a head defining an annular groove, the nut configured to extend at least partially over a tip of the screw.

8. The system of claim 7, wherein the nut includes a cylindrical portion extending away from the head of the nut, the cylindrical portion defining an opening configured to at least partially receive the tip of the screw.

9. The system of claim 7 or 8, further comprising a flexible nut washer distinct from the flexible washer, the flexible nut washer including an outer ring, at least one projection, and a slot at least partially separating the outer ring of the flexible nut washer from the at least one projection of the flexible nut washer, the at least one projection of the flexible nut washer configured to engage the annular groove of the nut.

10. The system of claim 9, wherein the at least one projection of the flexible nut washer includes three projections radially spaced around an inner hole of the flexible nut washer.

11. The system of claim 10, wherein the slot of the flexible nut washer includes three Y-shaped slots, each Y-shaped slot corresponding to a projection of the three projections.

12. The system of any of the preceding claims, further comprising an alignment insertion sleeve configured to connect the flexible washer to the screw.

13. The system of claim 12, further comprising an applicator configured to be removably attached to an end of the alignment insertion sleeve, the applicator including at least one projection configured to extend into an opening of the flexible washer to secure the flexible washer to the applicator.

14. The system of claim 12 or 13, wherein the alignment insertion sleeve includes a flange configured to removably engage with the at least one projection of the flexible washer.

15. The system of any of the preceding claims, wherein the projection is a radial leaf spring configured to be biased inward to retain the flexible washer to the annular groove.
